# EUROPÄISCHE PATENTANMELDUNG

(11) **EP 4 252 616 A1**
(43) Veröffentlichungstag der Anmeldung: **04.10.2023**
(21) Anmeldenummer: 23163006.2
(22) Anmeldetag: 21.03.2023
(51) Int. Cl.: A61B 1/00, A61B 1/05, A61M 25/00, G02B 23/24

(54) **ENDOSKOP UND VERFAHREN ZUR HERSTELLUNG EINER ABGEDICHTETEN EINHEIT EINES ENDOSKOPS**

(30) Priorität: 30.03.2022 US 202263325252 P
(71) Anmelder: OLYMPUS WINTER & IBE GMBH, 22045 Hamburg (DE)
(72) Erfinder: Wieters, Martin, 22885 Barsbüttel (DE); Nils, Torkuhl, 22045 Hamburg (DE); Mathias, Laser, 22391 Hamburg (DE)
(74) Vertreter: Noack, Andreas

(57) **Zusammenfassung**

Es wird ein Endoskop bereitgestellt, das einen Griff (3), einen länglichen Schaft (2) und eine in dem Schaft angeordnete Videokamera (4) umfasst, wobei das Endoskop eine Einheit (10) umfasst, die eine Mehrzahl von ineinander geschachtelten rohrförmigen Elementen (20, 30, 40) enthält, wobei die ineinander geschachtelten rohrförmigen Elemente (20, 30, 40) ein äußeres rohrförmiges Element (30, 40) und ein inneres rohrförmiges Element (20, 30) umfassen, die durch Kleben aneinander befestigt sind, wobei eine Bohrung (60, 61, 70) in dem äußeren rohrförmigen Element (30, 40) ausgebildet ist und ein Klebemittel in einem Spalt zwischen dem äußeren rohrförmigen Element (30, 40) und dem inneren rohrförmigen Element (20, 30) vorgesehen ist. Ferner wird ein Verfahren zur Herstellung einer Einheit von ineinandergeschachtelten rohrförmigen Elementen eines Endoskops bereitgestellt.

## Beschreibung

### Hintergrund

Die vorliegende Offenbarung bezieht sich auf ein Endoskop und ein Verfahren zur Herstellung einer abgedichteten Einheit eines Endoskops.

Endoskope werden seit vielen Jahren in der Chirurgie eingesetzt, um einen visuellen oder chirurgischen Zugang zu inneren Hohlräumen eines Patienten zu schaffen. Endoskope umfassen in der Regel einen Griff und einen langgestreckten Schaft. Je nach der beabsichtigten Anmeldung kann der Schaft flexibel oder starr sein.

In modernen Videoendoskopen ist eine Videokamera im Schaft, meist am distalsten Ende, angeordnet. Die Videokamera umfasst in der Regel eine Objektivlinse, einen Abbildungs- Chip zur Umwandlung eines von der Objektivlinse erzeugten Bildes in elektronische Signale und eine Verarbeitungsschaltung zur Verarbeitung der elektronischen Signale.

Aufgrund ihrer komplexen und teuren Konstruktion sind Videoendoskope in der Regel für den Mehrfachgebrauch vorgesehen und müssen daher einer sorgfältigen Aufbereitung unterzogen werden, damit eine Kreuzkontamination der Patienten mit Krankheitserregern verhindert werden kann. Eine solche Aufbereitung kann beinhalten, dass das Endoskop widrigen Bedingungen wie hohem Druck, hoher Feuchtigkeit und aggressiven Chemikalien ausgesetzt wird. Zum Schutz der empfindlichen optischen und elektronischen Komponenten des Endoskops vor solchen ungünstigen Bedingungen werden sie in einer separaten, abgedichteten Einheit mit einer Mehrzahl von ineinander geschachtelten rohrförmigen Elementen untergebracht. Diese rohrförmigen Elemente werden häufig durch Kleben miteinander verbunden. Eine solche abgedichtete Einheit wird manchmal auch als R-Unit bezeichnet.

Die Herstellung der versiegelten Einheit bringt einige Herausforderungen mit sich. Um die ineinander geschachtelten rohrförmigen Elemente miteinander zu verbinden, wird in der Regel eine Schicht eines Klebemittels auf die Außenfläche eines inneren rohrförmigen Elements aufgetragen und dann das innere rohrförmige Element in ein äußeres rohrförmiges Element geschoben. Dabei wird ein Teil des Klebemittels vom inneren rohrförmigen Element abgeschält und bildet Tropfen oder einen durchgehenden Strang an der Stirnfläche des äußeren rohrförmigen Elements. Dieses überschüssige Klebemittel muss entfernt werden, bevor der Herstellungsprozess fortgesetzt werden kann. Außerdem kann das Klebemittel zwischen dem inneren und dem äußeren rohrförmigen Element nicht mit UV-Strahlung bestrahlt werden, um ein schnelles Aushärten zu erreichen, so dass ein wärmehärtender Klebstoff verwendet werden muss. Wenn die rohrförmigen Elemente jedoch erhitzt werden, bevor sie fest verbunden sind, kann die Positionierung der rohrförmigen Elemente durch die Wärmeausdehnung beeinträchtigt werden.

Endoskope können auch andere Einheiten umfassen, die ineinander verschachtelte rohrförmige Elemente umfassen, die nicht unbedingt abgedichtet sein müssen. Für solche Einheiten können ähnliche Herausforderungen bei der Herstellung gelten.

Ziel der vorliegenden Offenbarung ist es, ein verbessertes Endoskop und ein verbessertes Verfahren zur Herstellung einer Einheit, wie z.B. einer abgedichteten Einheit, eines Endoskops bereitzustellen.

### Zusammenfassung der Offenlegung

Die vorliegende Offenbarung stellt ein Endoskop bereit, das einen Griff, einen länglichen Schaft und eine in dem Schaft angeordnete Videokamera umfasst, wobei das Endoskop eine Einheit mit einer Mehrzahl von ineinandergeschachtelten rohrförmigen Elementen umfasst, wobei die ineinandergeschachtelten rohrförmigen Elemente ein äußeres rohrförmiges Element und ein inneres rohrförmiges Element umfassen, die durch Kleben aneinander befestigt sind, wobei eine Bohrung in dem äußeren rohrförmigen Element ausgebildet ist und ein Klebemittel in einem Spalt zwischen dem äußeren rohrförmigen Element und dem inneren rohrförmigen Element vorgesehen ist. Die Bohrung in dem äußeren rohrförmigen Element erleichtert das Einspritzen des Klebemittels in den Spalt. Das Klebemittel kann in dem Spalt in einem die Bohrung umgebenden Bereich bereitgestellt werden. Das Klebemittel kann durch Kapillarwirkung im Spalt verteilt werden. Die Einheit kann eine abgedichtete Einheit der Videokamera des Endoskops sein.

Das innere rohrförmige Element kann eine Aussparung in einem Bereich umfassen, der der Bohrung des äußeren rohrförmigen Elements entspricht. Die Aussparung kann einen Hohlraum zwischen dem inneren rohrförmigen Element und dem äußeren rohrförmigen Element bilden, der durch die Bohrung injiziertes Klebemittel aufnehmen kann. Von dem Hohlraum aus kann das Klebemittel durch Kapillarwirkung leicht weiter in den Spalt verteilt werden. Nach dem Aushärten des Klebemittels kann das Klebemittel in dem Hohlraum eine formschlüssige Verbindung zwischen dem inneren rohrförmigen Element und dem äußeren rohrförmigen Element herstellen. Die Aussparung kann ein flacher Abschnitt, ein Einschnitt, eine Senkung oder ähnliches sein.

Entlang des Umfangs des äußeren rohrförmigen Elements kann eine Mehrzahl von Bohrungen angebracht sein, um eine bessere Verteilung des Klebemittels zu ermöglichen. Das Klebemittel kann eine spaltfreie Klebeschicht bilden, die sich vollständig um den Umfang des inneren rohrförmigen Elements erstreckt. Die spaltfreie Klebstoffschicht kann für eine sichere Befestigung und Abdichtung zwischen dem inneren rohrförmigen Element und dem äußeren rohrförmigen Element sorgen.

Die Mehrzahl der ineinander geschachtelten rohrförmigen Elemente kann ferner ein mittleres rohrförmiges Element umfassen, das zwischen dem äußeren rohrförmigen Element und dem inneren rohrförmigen Element angeordnet ist. In dem mittleren rohrförmigen Element können eine oder mehrere Bohrungen ausgebildet sein, so dass sich die Bohrungen des äußeren rohrförmigen Elements und die Bohrungen des mittleren rohrförmigen Elements überlappen. Der Begriff "Überlappung" ist hier so zu verstehen, dass er anzeigt, dass eine Bohrung des äußeren rohrförmigen Elements und eine entsprechende Bohrung des mittleren rohrförmigen Elements einen durchgehenden Durchgang bilden, der von einer Außenfläche des äußeren rohrförmigen Elements zu einer Außenfläche des inneren rohrförmigen Elements reicht.

Die Bohrungen des mittleren rohrförmigen Elements können einen kleineren Durchmesser haben als die Bohrungen des äußeren rohrförmigen Elements. Die Bohrungen des mittleren rohrförmigen Elements können gegenüber den Bohrungen des äußeren rohrförmigen Elements versetzt sein, so dass sich die Bohrungen des mittleren rohrförmigen Elements und die Bohrungen des äußeren rohrförmigen Elements teilweise überlappen. Bei teilweiser Überlappung der Bohrungen des mittleren rohrförmigen Elements und der Bohrungen des äußeren rohrförmigen Elements bildet sich zwischen dem inneren und dem äußeren rohrförmigen Element ein Hohlraum, aus dem das Klebemittel leicht in einen inneren Spalt zwischen dem inneren rohrförmigen Element und dem mittleren rohrförmigen Element und in einen äußeren Spalt zwischen dem mittleren rohrförmigen Element und dem äußeren rohrförmigen Element verteilt werden kann.

Die Bohrungen des mittleren rohrförmigen Elements können im Durchmesser größer sein als die Bohrungen des äußeren rohrförmigen Elements. Wenn die Bohrungen des mittleren rohrförmigen Elements größer sind als die Bohrungen des äußeren rohrförmigen Elements, entsteht zwischen dem inneren und dem äußeren rohrförmigen Element ein Hohlraum, aus dem das Klebemittel leicht in einen inneren Spalt zwischen dem inneren rohrförmigen Element und dem mittleren rohrförmigen Element und in einen äußeren Spalt zwischen dem mittleren rohrförmigen Element und dem äußeren rohrförmigen Element verteilt werden kann.

Das Klebemittel kann ein Klebemittel eines ersten Typs umfassen, das im Bereich mindestens einer ersten der Mehrzahl von Bohrungen aufgetragen wird, und ein Klebemittel eines zweiten Typs, das im Bereich mindestens einer zweiten der Mehrzahl von Bohrungen aufgetragen wird. Das Klebemittel des ersten Typs kann ein UV-härtendes Klebemittel sein. Das Klebemittel des zweiten Typs kann ein wärmehärtendes Klebemittel sein. Das Klebemittel des zweiten Typs kann eine höhere Glasübergangstemperatur aufweisen als das Klebemittel des ersten Typs. Das UV-härtende Klebemittel in der mindestens einen ersten Bohrung kann zunächst durch Bestrahlung mit UV-Strahlung ausgehärtet werden, wodurch die rohrförmigen Elemente aneinander befestigt werden. Anschließend kann das wärmehärtende Klebemittel durch Erwärmen der rohrförmigen Elemente ausgehärtet werden, während das zuvor ausgehärtete UV-härtende Klebemittel eine Depositionierung der rohrförmigen Elemente aufgrund von Wärmeausdehnung, Schrumpfung des Klebers oder ähnlichen Effekten verhindert.

Die vorliegende Offenbarung stellt ferner ein Verfahren zur Herstellung einer Einheit aus ineinander geschachtelten rohrförmigen Elementen eines Endoskops bereit, das die folgenden Schritte umfasst: Bereitstellen eines inneren rohrförmigen Elements und eines äußeren rohrförmigen Elements; Ausbilden einer oder mehrerer Bohrungen in dem äußeren rohrförmigen Element; Positionieren des inneren rohrförmigen Elements in dem äußeren rohrförmigen Element, so dass ein Spalt zwischen dem inneren und dem äußeren rohrförmigen Element gebildet wird; Aufbringen eines Klebemittels in die Bohrungen des äußeren rohrförmigen Elements; Ausbreiten des Klebemittels in dem Spalt durch Kapillarwirkung; und Aushärten des Klebemittels. Durch das Auftragen des Klebemittels nach dem Positionieren der rohrförmigen Elemente kann das Ablösen von überschüssigem Klebemittel vermieden werden, so dass bei der Herstellung der Einheit kein zusätzlicher Reinigungsschritt erforderlich ist.

Das Verfahren kann ferner die Schritte umfassen, eine oder mehrere Aussparungen an dem inneren rohrförmigen Element auszubilden und das innere rohrförmige Element in dem äußeren rohrförmigen Element so zu positionieren, dass die Aussparungen des inneren rohrförmigen Elements mit den Bohrungen in dem äußeren rohrförmigen Element übereinstimmen. Die Ausnehmungen können Hohlräume zwischen dem inneren rohrförmigen Element und dem äußeren rohrförmigen Element bilden. Die Ausnehmungen können Abflachungen, Einschnitte, Senkungen oder Ähnliches umfassen. Flache Abschnitte lassen sich leicht durch Fräsen auf der Außenfläche des inneren rohrförmigen Elements herstellen.

Das Verfahren kann ferner die folgenden Schritte umfassen: Bereitstellen eines mittleren rohrförmigen Elements; Ausbilden einer oder mehrerer Bohrungen in dem mittleren rohrförmigen Element; und Positionieren des mittleren rohrförmigen Elements und des inneren rohrförmigen Elements in dem äußeren rohrförmigen Element, so dass sich die Bohrungen des mittleren rohrförmigen Elements und die Bohrungen des äußeren rohrförmigen Elements überlappen.

Das mittlere rohrförmige Element kann so in dem äußeren rohrförmigen Element positioniert werden, dass die Bohrungen des mittleren rohrförmigen Elements gegenüber den Bohrungen des äußeren rohrförmigen Elements versetzt sind und sich nur teilweise mit den Bohrungen des äußeren rohrförmigen Elements überlappen.

Das Verfahren kann das Ausbilden einer Mehrzahl von Bohrungen in dem äußeren rohrförmigen Element und gegebenenfalls in dem mittleren rohrförmigen Element umfassen, wobei die Schritte des Auftragens, Ausbreitens und Aushärtens des Klebemittels das Auftragen eines Klebemittels eines ersten Typs auf mindestens eine erste der Bohrungen, das Aushärten des Klebemittels des ersten Typs, das Auftragen eines Klebemittels eines zweiten Typs auf mindestens eine zweite der Bohrungen, das Ausbreiten des Klebemittels des zweiten Typs in dem Spalt durch Kapillarwirkung und das Aushärten des Klebemittels des zweiten Typs umfassen.

Das Klebemittel des ersten Typs kann ein UV-härtendes Klebemittel sein, und das Aushärten des Klebemittels des ersten Typs kann die Bestrahlung des Klebemittels des ersten Typs mit UV-Strahlen beinhalten. Das Klebemittel des zweiten Typs kann ein wärmehärtendes Klebemittel sein, und das Aushärten des Klebemittels des zweiten Typs kann das Erhitzen des Klebemittels des zweiten Typs umfassen.

Einige Beispiele der vorliegenden Offenbarung werden im Folgenden anhand von illustrativen Zeichnungen beschrieben. Die beschriebenen Beispiele dienen dem besseren Verständnis und erheben keinen Anspruch auf Vollständigkeit oder darauf, den Geltungsbereich der beanspruchten Ansprüche in irgendeiner Weise einzuschränken.

### Kurze Beschreibung der Zeichnungen

### Die Zeichnungen zeigen:

- Fig. 1:: ein Endoskop,
- Fig. 2:: eine abgedichtete Einheit eines Endoskops in einem Längsschnitt,
- Fig. 3:: die abgedichtete Einheit nach Figur 2 in einem Querschnitt,
- Fig. 4:: ein Querschnitt einer weiteren abgedichteten Einheit.

### Ausführliche Beschreibung

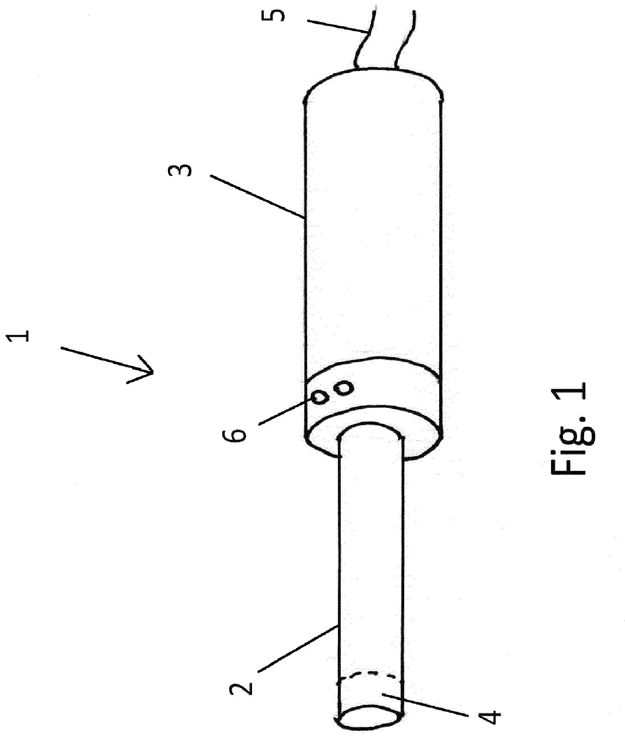

Abb. 1 zeigt ein Endoskop 1, das einen langgestreckten Schaft 2 und einen Griff 3 umfasst. Im distalen Bereich des Schafts 2 befindet sich eine Videokamera 4. Videosignale von der Videokamera 4 werden über ein Kabel 5 zur Verarbeitung, Anzeige und/oder Aufzeichnung an externe Videoverarbeitungsschaltungen (nicht dargestellt) weitergeleitet. Das Kabel 5 kann auch Strom und Beleuchtungslicht von einer externen Quelle (nicht abgebildet) an das Endoskop 1 liefern.

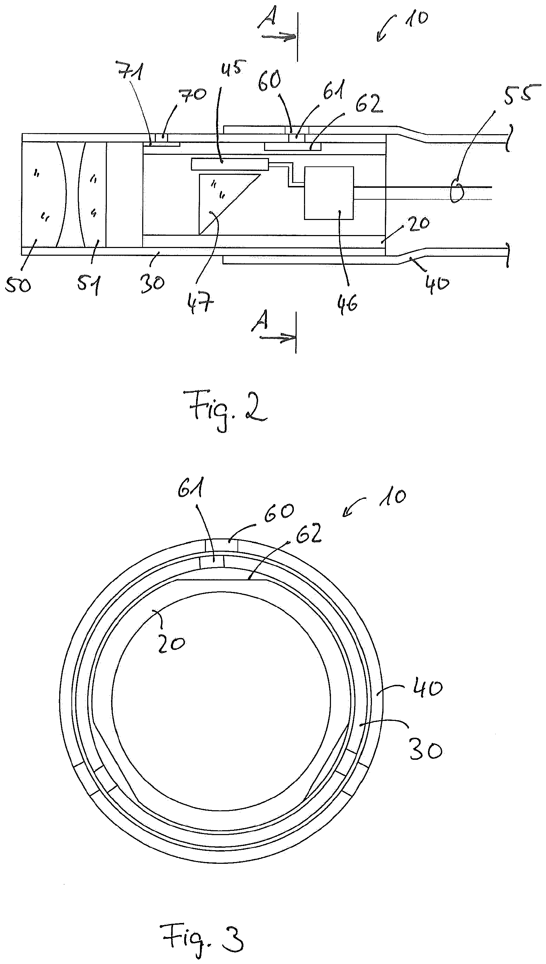

Zum Schutz der optischen und elektronischen Komponenten der Videokamera 4 vor schädlichen Umwelteinflüssen umfasst das Endoskop 1 eine abgedichtete Einheit 10, die in den Figuren 2 und 3 dargestellt ist. Figur 2 zeigt die abgedichtete Einheit 10 im Längsschnitt, Figur 3 zeigt die abgedichtete Einheit 10 im Querschnitt entlang der in Figur 2 dargestellten Ebene A - A.

Die abgedichtete Einheit 10 umfasst eine Mehrzahl von ineinander geschachtelten rohrförmigen Elementen 20, 30, 40. Ein inneres rohrförmiges Element 20 beherbergt einen Bildgebungschip 45, eine Vorverarbeitungsschaltung 46 und ein Prisma 47. Das innere rohrförmige Element wird manchmal auch als C-Halter bezeichnet. Ein mittleres rohrförmiges Element 30 nimmt das innere rohrförmige Element 20 und die Linsen 50, 51 eines Objektivsystems auf. Das mittlere rohrförmige Element 30 wird manchmal auch als des Objektivsystemrohr bezeichnet. Das äußere rohrförmige Element 40 nimmt das mittlere rohrförmige Element auf und erstreckt sich im Schaft 2 in proximaler Richtung. Verbindungsleitungen 55 erstrecken sich durch das äußere rohrförmige Element 40 zu einem Stopfen (nicht dargestellt), der das äußere rohrförmige Element 40 am proximalen Ende hermetisch abdichtet Das äußere rohrförmige Element 40 wird manchmal auch als Hauptrohr bezeichnet.

In der vorliegenden Ausführungsform sind die rohrförmigen Einheiten 20, 30, 40 mit einem runden Querschnitt dargestellt. Andere Querschnitte sind ebenfalls möglich, z. B. elliptisch, oval oder ähnliches. Die rohrförmigen Elemente 20, 30, 40 können auch prismatische Querschnitte aufweisen. Das innere rohrförmige Element 20 kann, was in Fig. 3 nicht dargestellt ist, eine innere Struktur aufweisen, die so eingerichtet ist, dass sie das Prisma 47 und den Abbildungschip 45 präzise hält.

Zur Herstellung der versiegelten Einheit 10 wird zunächst das innere rohrförmige Element 20 mit dem abbildenden Chip 45, dem Prisma 47 und der Vorverarbeitungsschaltung in das mittlere rohrförmige Element 30 mit den Linsen 50, 51 eingesetzt und sorgfältig so positioniert, dass ein Bild auf dem abbildenden Chip 45 scharf fokussiert wird. Dann werden das innere rohrförmige Element 20 und das mittlere rohrförmige Element 30 in das äußere rohrförmige Element 40 eingesetzt.

Die rohrförmigen Elemente 20, 30, 40 werden durch Verklebung aneinander befestigt. Dazu wird ein Klebemittel (nicht gezeigt) in die ringförmigen Spalte zwischen den jeweiligen rohrförmigen Elementen eingespritzt, wie in Fig. 3 dargestellt. Die Größe der Spalte in Abb. 3 ist nicht maßstabsgetreu, sondern zur besseren Sichtbarkeit übertrieben dargestellt.

Zum Aufbringen des Klebemittels sind Bohrungen 60 im äußeren rohrförmigen Element 40 und Bohrungen 61 im mittleren rohrförmigen Element vorgesehen, die sich gegenseitig überlappen. Ferner sind an der Außenfläche des inneren rohrförmigen Elements 20 Aussparungen 62 vorgesehen. Die Aussparungen 62 können die Form eines flachen Teils haben.

Die Bohrungen 60, 61 und die Aussparung 62 bilden einen Durchgang und einen Hohlraum, in den das Klebemittel eingespritzt wird. Nach der Injektion breitet sich das Klebemittel in den jeweiligen Zwischenräumen zwischen den rohrförmigen Elementen 20, 30, 40 durch Kapillarwirkung aus, d. h. das Klebemittel wird in die die Bohrungen 60, 61 umgebenden Zwischenräume gezogen. Durch eine geeignete Anzahl und Größe von Bohrungen 60, 61 und Aussparungen 62 am Umfang der abgedichteten Einheit bildet das Klebemittel eine durchgehende Schicht im Spalt, wodurch die rohrförmigen Elemente 20, 30, 40 sicher aneinander befestigt und abgedichtet werden.

Wenn Klebemittel eingespritzt wird, um den durch die Bohrungen 60, 61 und Aussparungen 62 gebildeten Hohlraum vollständig zu füllen, breitet sich das Klebemittel zunächst sowohl in einem inneren Spalt zwischen dem inneren rohrförmigen Element 20 und dem mittleren rohrförmigen Element 30 als auch in einem äußeren Spalt zwischen dem mittleren rohrförmigen Element 30 und dem äußeren rohrförmigen Element 40 aus. Nachdem etwas Klebemittel aus dem Hohlraum gezogen wurde, ist die Bohrung 60 nicht mehr mit Klebemittel gefüllt. Damit dennoch weiteres Klebemittel in den äußeren Spalt gezogen werden kann, sind die Bohrungen 60, 61 gegeneinander versetzt, so dass sie sich nur teilweise überlappen. Dadurch bildet sich zwischen einer Innenfläche des äußeren rohrförmigen Elements 40 und einer Außenfläche des inneren rohrförmigen Elements 20 ein Klebemittelreservoir, aus dem weiteres Klebemittel in den äußeren Spalt gesaugt werden kann. In der dargestellten Ausführungsform sind die Bohrungen 60 des äußeren rohrförmigen Elements im Durchmesser größer als die Bohrungen 61 des mittleren rohrförmigen Elements. Ein ähnlicher Effekt kann erreicht werden, wenn die Bohrungen 61 des mittleren rohrförmigen Elements 30 einen größeren Durchmesser aufweisen als die Bohrungen 60 des äußeren rohrförmigen Elements 40 (nicht dargestellt). Die flachen Abschnitte 62 dienen dazu, das Volumen des Aufnahmehohlraums für das Klebemittel weiter zu vergrößern, so dass mehr Klebemittel eingespritzt werden kann. Die Vertiefungen 62 sorgen außerdem für eine formschlüssige Verbindung zwischen dem mittleren rohrförmigen Element 30 und dem inneren rohrförmigen Element 20. Obwohl nicht dargestellt, können ähnliche Aussparungen an der Außenfläche des mittleren rohrförmigen Elements 30 vorgesehen sein.

Nach dem Auftragen des Klebemittels in den Zwischenräumen wird es zur Fixierung der rohrförmigen Elemente 20, 30, 40 ausgehärtet. Da das Klebemittel in den Zwischenräumen nicht mit UV-Licht bestrahlt werden kann, wird vorzugsweise ein wärmehärtendes Klebemittel verwendet. Als Klebemittel kann zum Beispiel ein Epoxidharz verwendet werden. Epoxidharze vertragen nach dem Aushärten die zu erwartenden hohen Temperaturen bei der Aufbereitung des Endoskops 1, z. B. im Autoklaven.

Zum Aushärten des wärmehärtenden Harzes muss die abgedichtete Einheit hohen Temperaturen ausgesetzt werden, bevor das Klebemittel vollständig ausgehärtet ist, was zu einer Verschiebung der jeweiligen rohrförmigen Elemente 20, 30, 40 führen und die optische Qualität des Endoskops 1 beeinträchtigen kann. Um eine solche Verschiebung zu verhindern, kann die Verklebung der rohrförmigen Elemente 20, 30, 40 in mehreren Schritten unter Verwendung verschiedener Arten von Klebemitteln erfolgen.

In einem ersten Schritt kann ein UV-härtender Klebstoff, z. B. ein Acrylharz, auf einen ersten Satz von Bohrungen 60, 61 und den entsprechenden flachen Teil aufgetragen werden. Der UV-härtende Klebstoff kann mit einer relativ hohen Viskosität oder Thixotropie aufgetragen werden, so dass er sich nicht wesentlich in die die erste Gruppe von Bohrungen 60, 61 umgebenden Lücken ausbreitet. Der UV-härtende Klebstoff kann dann durch Bestrahlung mit UV-Strahlung ausgehärtet werden, wodurch eine erste Klebeverbindung zwischen den rohrförmigen Elementen 20, 30, 40 entsteht.

In einem zweiten Schritt wird ein niedrigviskoses, wärmehärtendes Klebemittel auf die verbleibenden Bohrungen 60, 61 und Flachteile aufgetragen, so dass es sich durch Kapillarwirkung in die Zwischenräume ausbreitet. Nachdem sich das wärmehärtende Klebemittel vollständig ausgebreitet hat, werden die ineinander geschachtelten rohrförmigen Elemente in einem Ofen erhitzt, um das wärmehärtende Klebemittel vollständig auszuhärten und so die Klebeverbindung zu vervollständigen, während der zuvor ausgehärtete UV-härtende Klebstoff eine Verschiebung der jeweiligen rohrförmigen Elemente verhindert.

Der UV-härtende Klebstoff kann auf eine oder mehrere Bohrungen aufgetragen werden, die axial zu den Bohrungen 60, 61 versetzt sind, auf die das wärmehärtende Klebemittel aufgetragen wird. Wie in Fig. 2 gezeigt, kann in dem mittleren rohrförmigen Element 30 distal von einem distalen Ende des äußeren rohrförmigen Elements 40 eine zusätzliche Bohrung 70 vorgesehen sein, und an der Außenfläche des inneren rohrförmigen Elements 20 kann ein zusätzlicher flacher Abschnitt 71 vorgesehen sein. Der UV-härtende Klebstoff kann in den durch die Bohrung 70 und den flachen Abschnitt 71 gebildeten Hohlraum eingebracht und dann ausgehärtet werden, um das innere rohrförmige Element 20 und das mittlere rohrförmige Element 30 miteinander zu verbinden. Anschließend kann das wärmehärtende Klebemittel auf die Bohrungen 60, 61 und die Aussparungen 62 aufgetragen werden. Bei der anschließenden Aushärtung des wärmehärtenden Klebemittels durch Erhitzen verhindert die zuvor ausgehärtete Klebeverbindung zwischen dem inneren rohrförmigen Element 20 und dem mittleren rohrförmigen Element 30 eine Verschiebung dieser rohrförmigen Elemente, während eine mögliche Verschiebung des mittleren rohrförmigen Elements 20 im äußeren rohrförmigen Element40 für die optische Qualität des Endoskops 1 weit weniger kritisch ist.

Die Reihenfolge der oben genannten Schritte kann variiert werden. Beispielsweise kann der UV-härtende Klebstoff nach dem Einsetzen und Ausrichten des inneren und des mittleren rohrförmigen Elements 20, 30, des Abbildungschips 45, des Prismas 47 und der Linsen 50, 51, aber vor dem Einsetzen der so gebildeten Untereinheit in das äußere rohrförmige Element 40 aufgetragen und ausgehärtet werden.

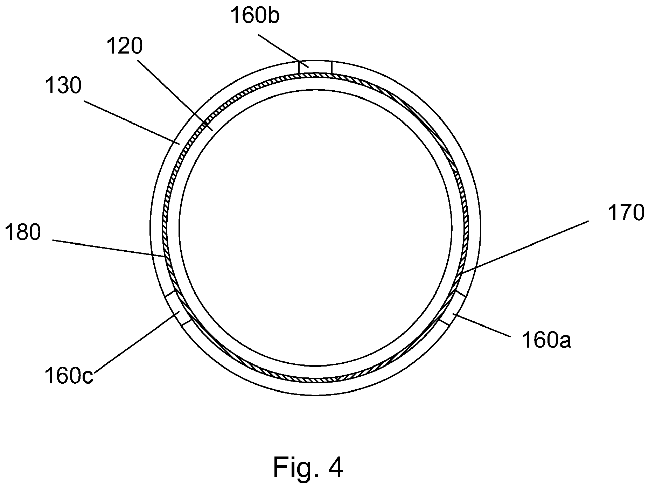

Abb. 4 zeigt eine Schnittdarstellung einer weiteren abgedichteten Einheit. Die in Fig. 4 dargestellte abgedichtete Einheit umfasst ein inneres rohrförmiges Element 120 und ein äußeres rohrförmiges Element 130, sowie einen Spalt zwischen dem inneren rohrförmigen Element 120 und dem äußeren rohrförmigen Element 130. Das äußere rohrförmige Element 130 umfasst entlang seines Umfangs drei Bohrungen 160a, 160b, 160c, die gleichmäßig über den Umfang verteilt sein können. Ein erster Klebstoff 170 füllt den Spalt in einem Bereich, der eine erste der Bohrungen 160a umgibt, und ein zweiter Klebstoff 180 füllt den Spalt in einem Bereich, der eine zweite und eine dritte der Bohrungen 160b, 160c umgibt. Bei dem ersten Klebstoff 170 kann es sich um einen UV-härtenden Klebstoff handeln. Bei dem zweiten Klebstoff 180 kann es sich um einen thermisch aushärtenden Klebstoff handeln.

Während die beschriebenen Ausführungsformen eine abgedichtete Einheit mit drei ineinander geschachtelten rohrförmigen Elementen 20, 30, 40 zeigen, kann auch eine andere Anzahl von rohrförmigen Elementen verwendet werden, z. B. zwei rohrförmige Elemente oder vier oder mehr rohrförmige Elemente. Die rohrförmigen Elemente können in einem einzigen Vorgang (der, wie oben beschrieben, zwei Klebeschritte umfassen kann) oder nacheinander geklebt werden. Beim aufeinanderfolgenden Verkleben der rohrförmigen Elemente kann zunächst ein innerstes Element mit einem zweitinneren Element verklebt werden. Dann kann das zweitunterste innere Element mit dem drittuntersten inneren Element verbunden werden, und so weiter. Ebenso kann ein äußerstes Element zuerst mit einem zweitäußersten äußeren Element verbunden werden, und dann wird das zweitäußerste äußere Element mit dem drittäußersten äußeren Element verbunden, und so weiter.

Weitere Ausführungsformen der Erfindung sind in den folgenden Beispielen dargestellt.

Beispiel 1. Endoskop, das einen Griff, einen länglichen Schaft und eine in dem Schaft angeordnete Videokamera umfasst,
wobei das Endoskop eine Einheit umfasst, die eine Mehrzahl von ineinander geschachtelten rohrförmigen Elementen enthält, wobei die ineinander geschachtelten rohrförmigen Elemente ein äußeres rohrförmiges Element und ein inneres rohrförmiges Element umfassen, die durch Kleben aneinander befestigt sind,
wobei eine Bohrung in dem äußeren rohrförmigen Element ausgebildet ist und ein Klebstoff in einem Spalt zwischen dem äußeren rohrförmigen Element und dem inneren rohrförmigen Element eingebracht ist.

Beispiel 2. Endoskop nach Beispiel 1, wobei die Einheit eine abgedichtete Einheit der Videokamera des Endoskops ist.

Beispiel 3. Endoskop nach Beispiel 1 oder 2, wobei das Klebemittel in dem Spalt in einem die Bohrung umgebenden Bereich eingebracht ist.

Beispiel 4. Endoskop nach einem der Beispiele 1 bis 3, wobei das innere rohrförmige Element eine Ausnehmung in einem Bereich umfasst, der der Bohrung des äußeren rohrförmigen Elements zugeordnet ist.

Beispiel 5. Endoskop nach einem der vorhergehenden Beispiele, wobei eine Mehrzahl von Bohrungen entlang eines Umfangs des äußeren rohrförmigen Elements ausgebildet ist.

Beispiel 6. Endoskop nach einem der vorangehenden Ansprüche, bei dem das Klebemittel eine spaltlose Klebeschicht bildet, die sich vollständig um den Umfang des inneren rohrförmigen Elements erstreckt.

Beispiel 7. Endoskop nach einem der vorhergehenden Beispiele, wobei die Mehrzahl der ineinandergeschachtelten rohrförmigen Elemente ferner ein mittleres rohrförmiges Element umfasst, das zwischen dem äußeren rohrförmigen Element und dem inneren rohrförmigen Element eingeschachtelt ist.

Beispiel 8. Endoskop nach Beispiel 7, wobei eine oder mehrere Bohrungen in dem mittleren rohrförmigen Element ausgebildet sind, so dass sich die Bohrungen des äußeren rohrförmigen Elements und die Bohrungen des mittleren rohrförmigen Elements überlappen.

Beispiel 9. Endoskop nach Beispiel 8, wobei die Bohrungen des mittleren rohrförmigen Elements einen kleineren Durchmesser haben als die Bohrungen des äußeren rohrförmigen Elements.

Beispiel 10. Endoskop nach Beispiel 8 oder 9, wobei die Bohrungen des mittleren rohrförmigen Elements gegenüber den Bohrungen des äußeren rohrförmigen Elements versetzt sind, so dass sich die Bohrungen des mittleren rohrförmigen Elements und die Bohrungen des äußeren rohrförmigen Elements teilweise überlappen.

Beispiel 11. Endoskop nach Beispiel 8, wobei die Bohrungen des mittleren rohrförmigen Elements einen größeren Durchmesser haben als die Bohrungen des äußeren rohrförmigen Elements.

Beispiel 12. Endoskop nach einem der Beispiele 5 bis 11, wobei das Klebemittel ein Klebemittel eines ersten Typs umfasst, das im Bereich von mindestens einer ersten der Mehrzahl von Bohrungen aufgebracht wird, und ein Klebemittel eines zweiten Typs, das im Bereich von mindestens einer zweiten der Mehrzahl von Bohrungen aufgebracht wird.

Beispiel 13. Endoskop nach Beispiel 12, wobei das Klebemittel der ersten Art ein UV-härtendes Klebemittel ist.

Beispiel 14. Endoskop nach Beispiel 12 oder 13, wobei das Klebemittel des zweiten Typs ein wärmehärtendes Klebemittel ist.

Beispiel 15. Endoskop nach einem der Beispiele 12 bis 14, wobei das Klebemittel des zweiten Typs eine höhere Glasübergangstemperatur aufweist als das Klebemittel des ersten Typs.

Beispiel 16. Verfahren zur Herstellung einer Einheit von ineinandergeschachtelten rohrförmigen Elementen eines Endoskops nach einem der Ansprüche 1 bis 15, das die folgenden Schritte umfasst:
- Bereitstellen eines inneren rohrförmigen Elements und eines äußeren rohrförmigen Elements;
- Ausbilden einer oder mehrerer Bohrungen in dem äußeren rohrförmigen Element;
- Positionieren des inneren rohrförmigen Elements (20, 30) in dem äußeren rohrförmigen Element, so dass ein Spalt zwischen dem inneren und dem äußeren rohrförmigen Element gebildet wird;

- Einbringen eines Klebemittels in die Bohrungen des äußeren rohrförmigen Elements;
- Ausbreiten des Klebemittels in dem Spalt durch Kapillarwirkung; und
- Aushärten des Klebemittels.

Beispiel 17. Verfahren nach Beispiel 16, umfassend ferner die folgenden Schritte:
- Ausbilden einer oder mehrerer Aussparungen an dem inneren rohrförmigen Element; und
- Positionieren des inneren rohrförmigen Elements in dem äußeren rohrförmigen Element, so dass die Aussparungen des inneren rohrförmigen Elements mit den Bohrungen in dem äußeren rohrförmigen Element übereinstimmen.

Beispiel 18. Das Verfahren nach Beispiel 16 oder 17 umfasst ferner die folgenden Schritte:
- Bereitstellen eines mittleren rohrförmigen Elements;
- Ausbilden einer oder mehrerer Bohrungen in dem mittleren rohrförmigen Element; und
- Positionieren des mittleren rohrförmigen Elements und des inneren rohrförmigen Elements in dem äußeren rohrförmigen Element, so dass die Bohrungen des mittleren rohrförmigen Elements und die Bohrungen des äußeren rohrförmigen Elements (40) überlappen.

Beispiel 19. Verfahren nach Beispiel 18, bei dem das mittlere rohrförmige Element in dem äußeren rohrförmigen Element so positioniert wird, dass die Bohrungen des mittleren rohrförmigen Elements gegenüber den Bohrungen des äußeren rohrförmigen Elements versetzt sind und sich nur teilweise mit den Bohrungen des äußeren rohrförmigen Elements überlappen.

Beispiel 20. Verfahren nach einem der Beispiele 16 bis 19, wobei eine Mehrzahl von Bohrungen in dem äußeren rohrförmigen Element und gegebenenfalls in dem mittleren rohrförmigen Element ausgebildet ist und wobei die Schritte des Einbringens, Verteilens und Aushärtens des Klebemittels Folgendes umfassen
- Einbringen eines Klebemittels eines ersten Typs in mindestens eine erste der Bohrungen;
- Aushärten des Klebemittels des ersten Typs;
- Einbringen eines Klebemittels eines zweiten Typs in mindestens eine zweite der Bohrungen;
- Ausbreiten des Klebemittels des zweiten Typs in dem Spalt durch Kapillarwirkung; und
- Aushärten des Klebemittels des zweiten Typs.

Beispiel 21. Verfahren nach Beispiel 18, wobei das Klebemittel des ersten Typs ein UV-härtendes Klebemittel ist und das Aushärten des Klebemittels des ersten Typs das Bestrahlen des Klebemittels des ersten Typs mit UV-Strahlung umfasst.

Beispiel 22. Verfahren nach Anspruch 18 oder 19, wobei das Klebemittel des zweiten Typs ein wärmehärtendes Klebemittel ist und das Aushärten des Klebemittels des zweiten Typs das Erhitzen des Klebemittels des zweiten Typs umfasst.

## Patentansprüche

1. Endoskop, das einen Griff (3), einen länglichen Schaft (2) und eine in dem Schaft angeordnete Videokamera (4) umfasst,
wobei das Endoskop eine Einheit (10) umfasst, die eine Mehrzahl von ineinander geschachtelten rohrförmigen Elementen (20, 30, 40) enthält, wobei die ineinander geschachtelten rohrförmigen Elemente (20, 30, 40) ein äußeres rohrförmiges Element (30, 40) und ein inneres rohrförmiges Element (20, 30) umfassen, die durch Kleben aneinander befestigt sind,
wobei eine oder mehrere entlang eines Umfangs des äußeren rohrförmigen Elements angeordnete Bohrungen (60, 61, 70) in dem äußeren rohrförmigen Element (30, 40) ausgebildet sind, und wobei ein Klebstoff in einem Spalt zwischen dem äußeren rohrförmigen Element (30, 40) und dem inneren rohrförmigen Element (20, 30) eingebracht ist.

2. Endoskop nach Anspruch 1, wobei die Einheit (10) eine abgedichtete Einheit der Videokamera (4) des Endoskops ist.

3. Endoskop nach Anspruch 1 oder 2, wobei das Klebemittel in dem Spalt in einem die Bohrung (60, 61, 70) umgebenden Bereich eingebracht ist, und vorzugsweise eine spaltlose Klebeschicht bildet, die sich vollständig um den Umfang des inneren rohrförmigen Elements (20, 30) erstreckt.

4. Endoskop nach einem der Ansprüche 1 bis 3, wobei das innere rohrförmige Element (20) eine Ausnehmung (62, 71) in einem Bereich umfasst, der der Bohrung (60, 70) des äußeren rohrförmigen Elements zugeordnet ist.

5. Endoskop nach einem der vorhergehenden Ansprüche, wobei die Mehrzahl der ineinandergeschachtelten rohrförmigen Elemente (20, 30, 40) ferner ein mittleres rohrförmiges Element (30) umfasst, das zwischen dem äußeren rohrförmigen Element (40) und dem inneren rohrförmigen Element (20) eingeschachtelt ist.

6. Endoskop nach Anspruch 5, wobei eine oder mehrere Bohrungen (61) in dem mittleren rohrförmigen Element (30) ausgebildet sind, so dass sich die Bohrungen (60) des äußeren rohrförmigen Elements (40) und die Bohrungen (61) des mittleren rohrförmigen Elements (30) überlappen.

7. Endoskop nach Anspruch 6, wobei die Bohrungen (61) des mittleren rohrförmigen Elements (30) gegenüber den Bohrungen (60) des äußeren rohrförmigen Elements (40) versetzt sind, so dass sich die Bohrungen (61) des mittleren rohrförmigen Elements (30) und die Bohrungen (60) des äußeren rohrförmigen Elements (40) teilweise überlappen.

8. Endoskop nach einem der Ansprüche 1 bis 7, wobei das Klebemittel ein Klebemittel eines ersten Typs umfasst, das im Bereich von mindestens einer ersten der Mehrzahl von Bohrungen (60, 61, 70) aufgebracht wird, und ein Klebemittel eines zweiten Typs, das im Bereich von mindestens einer zweiten der Mehrzahl von Bohrungen (60, 61, 70) aufgebracht wird, wobei vorzugsweise
- das Klebemittel der ersten Art ein UV-härtendes Klebemittel ist,
- das Klebemittel des zweiten Typs ein wärmehärtendes Klebemittel ist, und/oder
- das Klebemittel des zweiten Typs eine höhere Glasübergangstemperatur aufweist als das Klebemittel des ersten Typs.

9. Verfahren zur Herstellung einer Einheit von ineinandergeschachtelten rohrförmigen Elementen eines Endoskops nach einem der Ansprüche 1 bis 15, das die folgenden Schritte umfasst:
- Bereitstellen eines inneren rohrförmigen Elements (20, 30) und eines äußeren rohrförmigen Elements (30, 40);
- Ausbilden einer oder mehrerer Bohrungen (60, 61, 70) in dem äußeren rohrförmigen Element (30, 40);
- Positionieren des inneren rohrförmigen Elements (20, 30) in dem äußeren rohrförmigen Element (30, 40), so dass ein Spalt zwischen dem inneren und dem äußeren rohrförmigen Element (20, 30, 40) gebildet wird;
- Einbringen eines Klebemittels in die Bohrungen (60, 61, 70) des äußeren rohrförmigen Elements (30, 40);
- Ausbreiten des Klebemittels in dem Spalt durch Kapillarwirkung; und
- Aushärten des Klebemittels.

10. Das Verfahren nach Anspruch 9 umfasst ferner die folgenden Schritte:
- Ausbilden einer oder mehrerer Aussparungen (62, 71) an dem inneren rohrförmigen Element (20); und
- Positionieren des inneren rohrförmigen Elements (20) in dem äußeren rohrförmigen Element (30), so dass die Aussparungen (62, 71) des inneren rohrförmigen Elements (20) mit den Bohrungen (61) in dem äußeren rohrförmigen Element (30) übereinstimmen.

11. Das Verfahren nach Anspruch 9 oder 10 umfasst ferner die folgenden Schritte:
- Bereitstellen eines mittleren rohrförmigen Elements (30);
- Ausbilden einer oder mehrerer Bohrungen (61) in dem mittleren rohrförmigen Element (30); und
- Positionieren des mittleren rohrförmigen Elements (30) und des inneren rohrförmigen Elements (20) in dem äußeren rohrförmigen Element (40), so dass die Bohrungen (61) des mittleren rohrförmigen Elements (30) und die Bohrungen (60) des äußeren rohrförmigen Elements (40) überlappen.

12. Verfahren nach Anspruch 11, bei dem das mittlere rohrförmige Element (30) in dem äußeren rohrförmigen Element (40) so positioniert wird, dass die Bohrungen (61) des mittleren rohrförmigen Elements (30) gegenüber den Bohrungen (60) des äußeren rohrförmigen Elements (40) versetzt sind und sich nur teilweise mit den Bohrungen (60) des äußeren rohrförmigen Elements (40) überlappen.

13. Verfahren nach einem der Ansprüche 9 bis 12, wobei eine Mehrzahl von Bohrungen (60, 70) in dem äußeren rohrförmigen Element (30, 40) und gegebenenfalls in dem mittleren rohrförmigen Element (30) ausgebildet ist und wobei die Schritte des Einbringens, Verteilens und Aushärtens des Klebemittels Folgendes umfassen
- Einbringen eines Klebemittels eines ersten Typs in mindestens eine erste der Bohrungen (60, 70);
- Aushärten des Klebemittels des ersten Typs;
- Einbringen eines Klebemittels eines zweiten Typs in mindestens eine zweite der Bohrungen (60);
- Ausbreiten des Klebemittels des zweiten Typs in dem Spalt durch Kapillarwirkung; und
- Aushärten des Klebemittels des zweiten Typs.

14. Verfahren nach Anspruch 13, wobei das Klebemittel des ersten Typs ein UV-härtendes Klebemittel ist und das Aushärten des Klebemittels des ersten Typs das Bestrahlen des Klebemittels des ersten Typs mit UV-Strahlung umfasst.

15. Verfahren nach Anspruch 13 oder 14, wobei das Klebemittel des zweiten Typs ein wärmehärtendes Klebemittel ist und das Aushärten des Klebemittels des zweiten Typs das Erhitzen des Klebemittels des zweiten Typs umfasst.
